# EUROPEAN PATENT SPECIFICATION

(11) **EP 1 519 656 B1**
(45) Date of publication and mention of the grant of the patent: **28.06.2006**
(21) Application number: 03707936.5
(22) Date of filing: 18.02.2003
(51) Int. Cl.: A23L 2/42, A61L 2/18, B08B 9/08

(54) **A METHOD OF ASEPTICALLY STORING A BULK STERILE PRODUCT**
VERFAHREN ZUM ASEPTISCHEN LAGERN EINER GROSSMENGE EINES STERILEN PRODUKTS
PROCEDE PERMETTANT DE STOCKER EN MILIEU ASEPTIQUE UN PRODUIT STERILE EN VRAC

(30) Priority: 19.02.2002 BR 0200434
(43) Date of publication of application: 06.04.2005
(73) Proprietor: Fischer S.A. Agroindustria, CEP-15990-020 Matao, SP (BR)
(72) Inventor: MORAES, Ricardo, Ermirio de, 15990-902- Matao- SP (BR)
(74) Representative: Scott, Susan Margaret
(86) International application number: PCT/BR2003/000021
(87) International publication number: WO 2003/070024

(56) References cited:
- DE-A- 2 401 764
- US-A- 3 096 181
- US-A- 3 918 942
- US-A- 4 287 821
- US-A- 6 098 672
- US-B1- 6 277 328
- US-B1- 6 326 032

## Description

### Field of the Invention

The present invention relates to a method of aseptically storing and transporting bulk sterile products, particularly liquid food products such as citrus juices "in natura" for long periods of storage and long-distance transportation by road, fluvial and marine systems.

### Background of the Invention

In processing bulk food products to be transported to the final consumer it may be necessary to store a large amount of the product in storage tanks for a long period of time and under aseptic conditions in order to guarantee the quality thereof. This period of time may exceed one year.

In order to meet the above-cited conditions, it is imperative to prevent the introduction of any contaminating microorganism in the storage tanks, even if in minute amounts since it may be sufficient to contaminate the whole content of the tank.

The constant maintenance of the temperature within ideal parameters according to the characteristics of the product may be of the utmost importance to guarantee the quality of the stored product.

In the last few years, several methods and equipment have been developed specially aiming at the aseptic storage of bulk food products for long periods of time.

Sterilization methods using large amounts of sterilizing chemical liquids generate an expressive negative impact on the environment, as is the case of the method proposed in patents US 6,030,580 and US 6,277,328. Those prior art documents disclose methods for aseptically storing and transporting sterile food products wherein the container in which said food product will be stored must be flooded with a chemical sterilizing liquid. According to the teachings of those documents, it is essential that the whole internal area of tank or container is flooded. Preferably even the tank vents should also be flooded so as to ensure that there will be no microorganism contamination.

US-A-3096181 and US-A-6326032 discloses further sterilization methods wherein the sterilizing solution is sprayed into the container.

Maintaining the asepsis conditions of food products represents a considerable difficulty when said products are stored for long periods and/or transported over long distances. The considerable difficulty relies on preventing microbiological contamination due to the high cost and complexity of the known technology available for providing such a guarantee.

Citrus products, for example, require a severe control in terms of temperature and sterilization during the storage and transportation processes in order to avoid the need of repeating thermal treatment operations, since such repetitive operations leads to a loss of quality of the product.

### Objectives of the Invention

An objective of the present invention is to provide a method for aseptically storing a bulk product, particularly a sterilized liquid product to be stored in large amounts and allowing same to be transported over long distances by road, fluvial and marine systems, without using large amounts of sterilizing chemical products that are harmful to the environment and without requiring complex and expensive procedures and installations of difficult feasibility.

Another objective of the present invention is to provide a method as defined above and that allows the maintenance of the bulk product stored in a container such as a tank with an internal environment kept in sterile condition.

### Summary of the Invention

In order to achieve the above objectives,the present invention provides a method of aseptically storing a bulk sterile product in stationary storing tanks or containers designed for transporting said sterile product, according to claim 1.

The method according to the present invention now makes it possible to sterilize a storage tank without the need of flooding it with a sterilizing chemical liquid as it was known from the prior art. In the present method the sterilization of the tank is carried out by applying the chemical sterilizing agent in a way to ensure that all inner surfaces of the tanks are covered by said agent, but the internal volume thereof are not completely filled with it. It can be achieved by spraying high-pressure jets against the inner surfaces with only a small fraction of the amount of the chemical sterilizing agent that would be required in a method comprising a flooding step.

The addition of the sterilizing liquid is carried out after the step for cleaning the inside of the container. After draining the sterilizing liquid the container is completely filled with a sterile liquid, for example pasteurized water, in order to expel, to the atmosphere the whole air that is inside said container. Then, the container is sealed and pressurized with an inert gas for expelling said sterile liquid and creating an inert-gas atmosphere inside the container. The bulk sterile product may then be fed to the container, as described above with respect to the preceding variant.

### Brief Description of the Drawings

The invention will now be described in further detail with reference to the attached drawings, as follows:

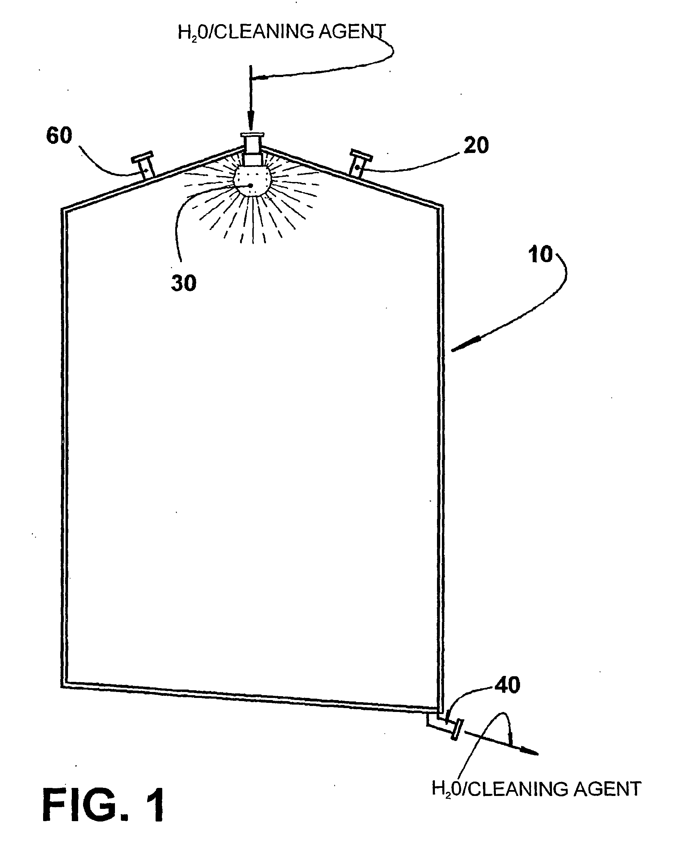

- Figure 1 represents a schematic vertical cross-section view of a tank for aseptically storing a sterile product, which is subjected to a cleaning operation by internally spraying water and cleaning agents;

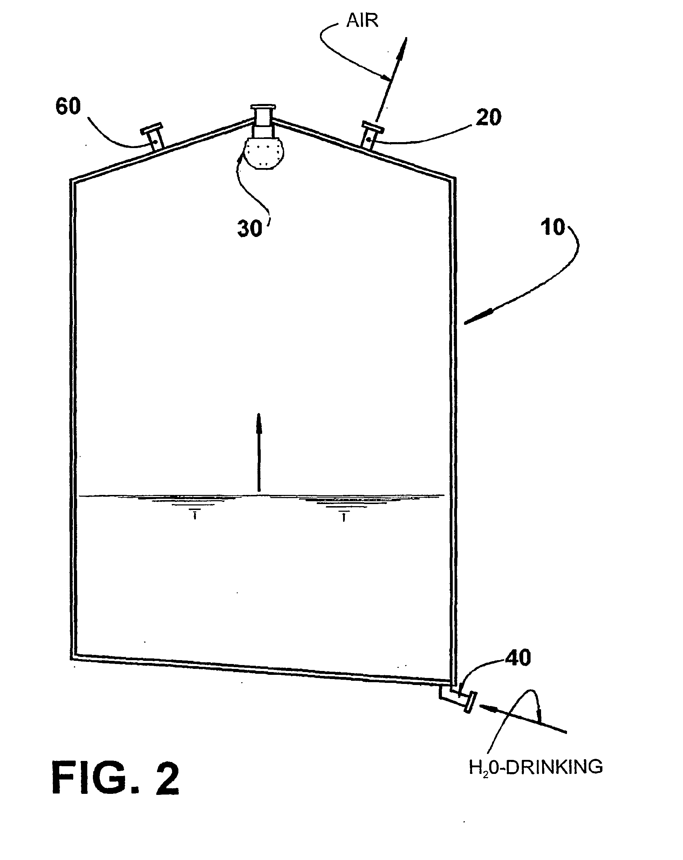

- Figure 2 represents a schematic vertical cross-section view of a tank for aseptically storing a sterile product, which is subjected to flooding with drinking-water to expel air out of it;

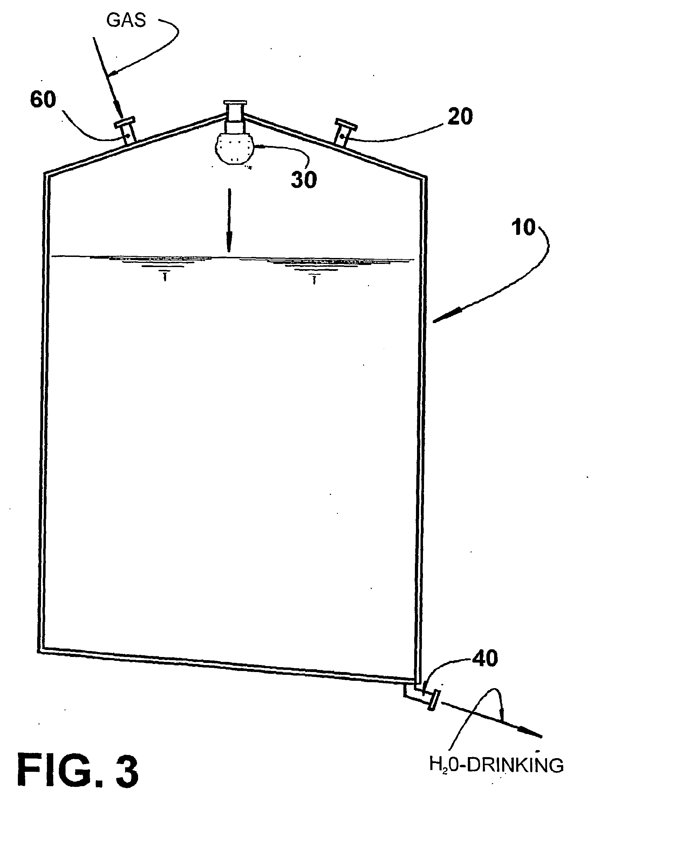

- Figure 3 represents a view similar to that of figure 2, but illustrating the phase of expelling the water by injecting an inert gas into the tank;

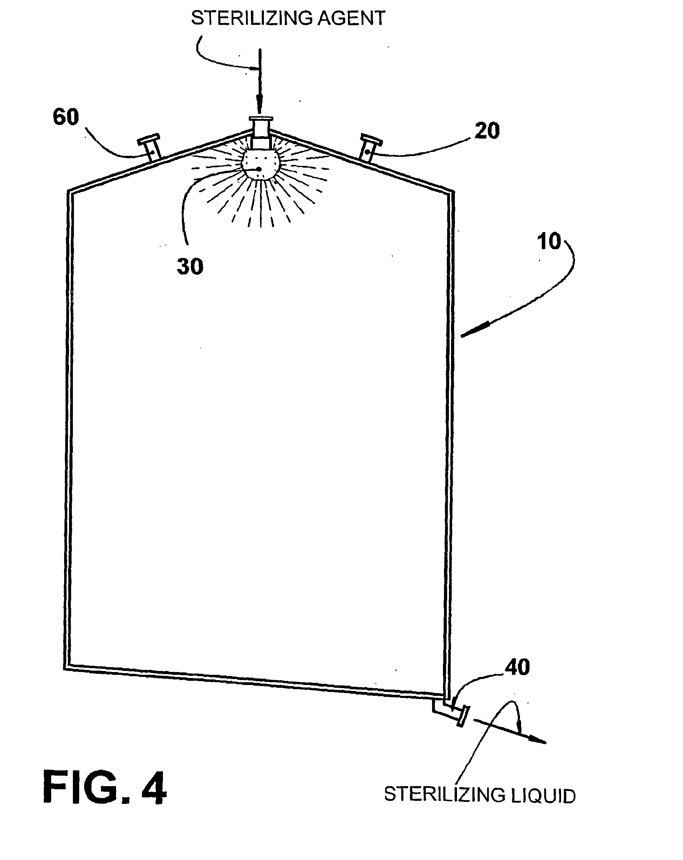

- Figure 4 represents a view similar to that of figures 1-3, but illustrating the tank totally filled with a pressurized inert gas and being entirely sprayed with a sterilizing chemical liquid, which is drained at the lower portion of the tank; and

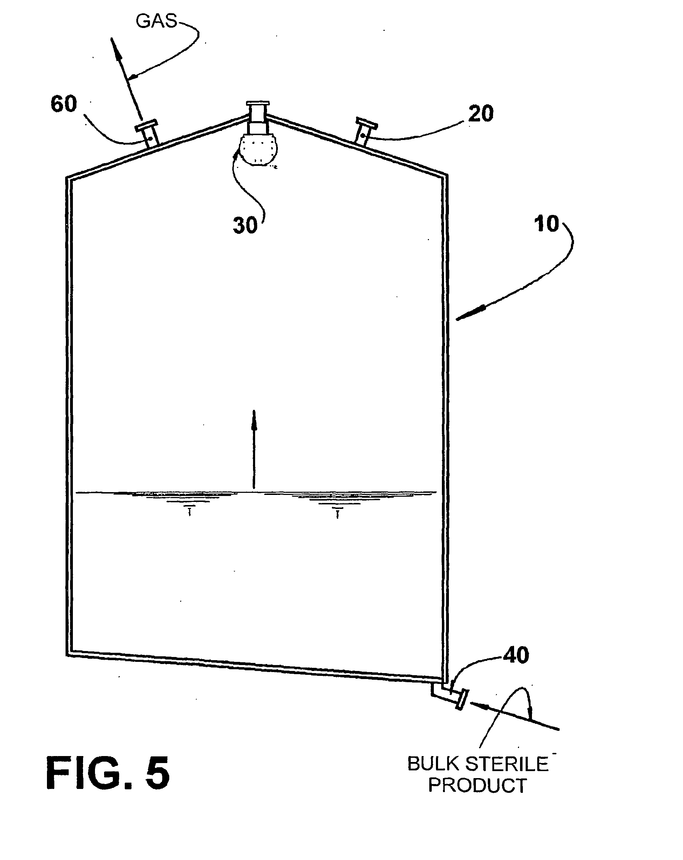

- Figure 5 represents a view similar to those of the preceding figures, but illustrating the tank while it is being filled with the bulk sterile product.

### Detailed Description of the Invention

The method of the invention as defined above in a generic way permits to store large amounts of bulk sterile products, such as orange juice "in natura", for long periods of time and transport to long distances, whereby those products can be maintained in storage tanks located close to the place where the products are extracted or even located at considerable distances from the extraction site. This being the case, the products are stored in tanks for transportation by road, fluvial and marine systems, etc. associated to adequate installations and under controlled operational conditions, with a view to maintain the high quality of the products in terms of flavor and original properties to be offered to the consumer market.

Although the present invention may be used for transporting different kinds of products that require aseptic storage and transport environments as in the case of pharmaceutical products or the like, it is particularly useful for aseptically storing and transporting liquid food products such as fruit juices, maintained under certain conditions, such as temperature and sterility.

The present invention is especially suitable for storing and transporting orange juice that has been pasteurized and kept in liquid state at a temperature that usually does not exceed 5°C.

The method of the invention comprises basic steps of sterilizing a storage or transport container 10 with sterilizing chemical products, in a pressurized inert-gas atmosphere during the cleaning/sterilizing operation.

Thus, during sterilization of the container 10 sterilizing agents that are duly approved for use on food products, such as oxonia or iodophorm are employed.

According to the illustrated embodiment, the method or the present invention is used to provide an aseptic storage of a liquid edible product in a stationary storage container or tank 10, built from any suitable material such as stainless steel and provided with one or more upper vents 20 for expelling the air contained inside the tank 10 at the beginning of the sterilization process. A spraying device (spray ball) 30 is placed at the upper portion of the tank 10 to provide high-pressure jets of chemical cleaning agents, rinsing water and sterilizing liquid during different phases of the sterilizing process as will be described below.

The spraying device 30 may be arranged in any suitable system, provided that it is positioned inside the tank 10 so as to spray high-pressure jets against all the inner surfaces of the tank 10.

At the lower part of the tank 10, a lower nozzle 40 is provided for inlet and outlet of the liquid product to be stored as well as the potable water used in one of the stages of the sterilizing process. This lower nozzle is further used as a drain for the sterilizing liquid to be introduced in a reservoir (not shown) and that may be recycled into the tank 10 through the spraying device 30 during the sterilizing step, as will be described below. At the upper part the tank 10 is provided with an injection inlet 60 for its pressurization with an inert gas.

It should be understood that container 10 may have different shapes depending on the specific function to be performed. For example, if it is intended for transporting of the product, it will have a shape considered to be the most adequate to the transportation means on which it will be installed. Preferably tank 10 is further externally covered by an adequate thermal insulation (not shown) whenever it is necessary to keep a certain controlled temperature inside the tank 10, as in the case of citrus juices.

The method of the invention is initiated by a step of cleaning the internal area of tank 10 by applying chemical cleaning agents to all its inner surfaces. The chemical cleaning agent may comprise a caustic soda solution heated up to about 75°C with a concentration of about 1.5%, or a phosphoric acid solution with a pH ranging from about 3 to 4, or mixtures thereof.

The cleaning agents are preferably applied by spraying jets inside the tank from the spraying device 30 ("spray ball"), adequately positioned to reach all the inner surfaces of the tank 10.

It should be further understood that the cleaning step may be carried out with other agents than the above exemplified ones, as long as they are suitable for removing impurities out of the tank 10, which will later be sterilized and filled with an edible product, for example.

Also, the application of chemical cleaning agents may be carried out by other procedures, provided that they meet with the cleaning requirements of the specific method in question.

In a preferred embodiment of the invention, the internal cleaning operation of tank 10 also comprises an initial rinsing step with water, generally at room temperature, and then a step comprising applying a first chemical cleaning agent exemplified by the heated caustic soda solution. A further water-rinsing operation is carried out, followed by the application of a second cleaning agent, for example, the phosphoric acid solution. Then, right after the cleaning step of the tank using at least one chemical cleaning agent, all inner surfaces of the tank are subjected to the application of a sterilizing liquid.

The container is subjected to a spray application of a sterilizing liquid against its inner surfaces in order completely to sterilize the internal area of the tank 10.

According to the invention, the application of the sterilizing liquid is effected by jetting from a spraying device 30 arranged so as to provide high-pressure jets of the sterilizing liquid against the inner surfaces of tank 10, which should preferably be built in such a way that its inner area can be totally subjected to this sterilizing method.

It is recommendable that the geometry of the components implanted in the lower part of the tank are suitable for effectively receiving the flow of sterilizing liquid from the spraying device 30 (spray ball). However, even if it cannot be ensured that the lower geometry of the tank is adequate, the claimed method would still be effective provided that there is accumulation of certain amount of the sterilizing solution delivered by the spray ball up to the level of immersion of those components that have not been effectively sprayed.

The sterilizing liquid may comprise a 0.3% oxonia solution or a iodophorm solution at 25 ppm, or any other conventional sterilizing solution appropriate for process of sterilizing containers or environments in which sterile perishable products will be stored.

After the sterilization step the used sterilizing liquid is drained and adequately collected through the lower outlet nozzle 40 and may be reused in a subse-quent sterilizing operation. After the sterilizing liquid is drained out from the tank the latter can be completely filled with a sterile liquid such as pasteurized water, in order to expel all the air contained in the tank 10 to the atmosphere. Tank 10 is then sealed and pressurized with inert gas, the sterile liquid contained in the tank being expelled, and thus forming therein a pressurized inert gas atmosphere that allows the tank to be fed with the bulk sterile produc

According to a preferred embodiment of the invention, the inert gas may be, for example, nitrogen, carbon dioxide or argon, usually injected from the upper part of the tank through the injection inlet 60 at a positive pressure ranging from 10 to 26 mbar.

Following the pressuring step, the bulk sterile product is fed to the tank 10, occupying at least a part of its useful internal volume while keeping the inert atmosphere pressurized. The bulk sterile product may be fed to tank 10, for instance, through the lower nozzle 40, which may also be used as an outlet for that product and for feeding and draining the water used in the cleaning and sterilizing steps.

After the product has been fed, tank 10 is sealed and maintained with its internal atmosphere with pressurized inert gas and at a temperature required for preserving the stored product.

The above described method when applied, for example, to an ordinary tank used for aseptically storing and/or transporting a sterilized liquid food product results in considerable handling economy since there is no need to effect more than one thermal treatment of the product until the delivery to the end client, even if long storage periods or long distances travels are involved (only a conventional thermal sterilization treatment prior to the storage method of the invention would be necessary).

In addition to the above mentioned advantages, the present method for aseptically storing and transporting a product has at least two further important aspects with regard to the technical solutions known from the prior art. Firstly, a sterilization step by means of a spraying device (spray ball) guarantees that all inner surfaces of the tank be sanitized and sterilized due to the radial and centered distribution of the sterilizing agents from above and, secondly, a significant environmental gain is achieved due to the reduced volume of chemical agents employed in that sterilization step.

The present invention has been described with reference to only one preferred embodiment directed to storing and/or transporting sterilized and bulk liquid food products with a view to decrease the number of steps and processes of microbiological control which are usually required during the periods of storing and transporting said products, and also to reduce the costs involved with those steps. However, any person skilled in the art would promptly understand that several process parameters and details may be modified made without departing from the spirit and protection scope of the present invention.

## Claims

1. A method of aseptically storing a bulk sterile product in stationary storing tanks or containers (10) designed for transporting said sterile product, **characterized by** comprising the steps of:
a) cleaning the stationary storing tank or container (10) by applying at least one chemical cleaning agent to its inner surfaces;
b) applying a liquid sterilizing agent by means of high pressure jet stream to all inner surfaces of the cleaned stationary storing tank or container (10) in such a manner that the stationary storing tank or container (10) is not flooded with said liquid sterilizing agent;
c) draining the liquid sterilizing agent applied to the inner surfaces of the stationary storing tank or container (10).
d) completely filling the sterilized stationary storing tank or container (10) with a sterile liquid to expel all the air contained in it out to the atmosphere;
e) sealing and pressurizing the stationary storing tank or container (10) with an inert gas, expelling the sterile liquid out of it and creating a pressurized inert gas atmosphere inside the stationary storing tank or container (10),
f) feeding an amount of a sterile product into the sterilized stationary storing tank or container wherein said sterile product occupies at least a portion of the stationary storing tank or container internal volume, while keeping the pressurized inert atmosphere; and
g) sealing the sterilized stationary storing tank or container (10) comprising the sterile product so as to maintain the pressurized inert gas atmosphere inside it.

2. A method according to claim 1, **characterized in that** the sterile liquid used in step (c) is pasteurized water.

3. A method according to claim 1, **characterized in that** the bulk sterile product comprises fruit juice.

4. A method according to claim 1, **characterized in that** the chemical cleaning is selected from the group consisting of phosphoric acid solution with a pH ranging from 3 to 4, a caustic soda solution heated up to about 75°C, or mixtures thereof.

5. A method according to claim 1, **characterized in that** the inert gas is nitrogen.

6. A method according to claim 1, **characterized in that** the inert gas is carbon dioxide.

7. A method according to claim 1, **characterized in that** the inert gas is argon.

8. A method according to claim 1, **characterized in that** the positive pressure of the inert gas is of about 10 to 26 mbar.

9. A method according to claim 1. **characterized in that** the sterilizing agent comprises an oxonia solution.

10. A method according to claim 1, **characterized in that** the sterilizing agent comprises an idophorm solution.

11. A method according to claim 1, **characterized in that** the cleaning step (a) comprises the following steps:
a1) internally rinsing the stationary storing tank or container (10) with potable water;
a2) applying a caustic soda solution heated up to about 75°C to the inner surfaces of the stationary storing tank or container (10); and
a3) internally rinsing the stationary storing tank or container (10) treated in step (a2) with potable water and applying a second cleaning agent such as a phosphoric acid solution wit a pH of about 3 to 4.

12. A method according to claim 1, **characterized in that** the steps of cleaning and sterilizing the stationary storing tank or container (10) are carried out by spraying high-pressure jets against the inner surfaces of the said container (10), wherein said high-pressure jets are delivered from a spraying device (30) internally mounted at the upper portion of the container (10).

13. A method according to claim 1, **characterized in that** the sterile product to be stored is introduced into the stationary storing tank or container (10), while a corresponding volume of inert gas is removed.

## Patentansprüche

1. Verfahren zum aseptischen Lagern einer Großmenge eines sterilen Produkts in stationären Lagertanks oder -behältern (10), die zum Transportieren des sterilen Produkts ausgebildet sind, **gekennzeichnet durch** die Schritte:
a) Reinigen des stationären Lagertanks oder - behälters (10) **durch** Aufbringen zumindest eines chemischen Reinigungsmittels auf seine Innenflächen;
b) Aufbringen eines flüssigen sterilisierenden Mittels **durch** einen Hochdruck-Strahlstrom auf alle inneren Oberflächen des gereinigten stationären Lagertanks oder -behälters (10) in einer solchen Weise, dass der stationäre Lagertank oder -behälter (10) nicht mit dem flüssigen sterilisierenden Mittel überflutet wird;
c) Abziehen des flüssigen sterilisierenden Mittels, das auf die inneren Oberflächen des stationären Lagertanks oder -behälters (10) aufgebracht wurde;
d) vollständiges Füllen des sterilisierten stationären Lagertanks oder -behälters (10) mit einer sterilen Flüssigkeit, um die gesamte in ihm enthaltene Luft in die Atmosphäre auszutreiben;
e) Abdichten und Unterdrucksetzen des stationären Lagertanks oder -behälters (10) mit einem inerten Gas, Austreiben der sterilen Flüssigkeit aus diesem und Schaffen einer Druckatmosphäre aus inertem Gas innerhalb des stationären Lagertanks oder -behälters (10);
f) Hineinführen einer Menge eines sterilen Produkts in den sterilisierten stationären Lagertank oder -behälter, worin das sterile Produkt zumindest einen Teil des inneren Volumens des stationären Lagertanks oder -behälters einnimmt, wobei die inerte Druckatmosphäre aufrechterhalten wird; und
g) Abdichten des sterilisierten stationären Speichertanks oder -behälters (10), der das sterile Produkt aufweist, derart, dass die Druckatmosphäre aus inertem Gas innerhalb von diesem aufrechterhalten wird.

2. Verfahren nach Anspruch 1, **dadurch gekennzeichnet, dass** die im Schritt (c) verwendete sterile Flüssigkeit pasteurisiertes Wasser ist.

3. Verfahren nach Anspruch 1, **dadurch gekennzeichnet, dass** die Großmenge eines sterilen Produkts Fruchtsaft aufweist.

4. Verfahren nach Anspruch 1, **dadurch gekennzeichnet, dass** die chemische Reinigung ausgewählt ist aus der Gruppe besteht aus Phosphorsäurelösung mit einem pH-Wert im Bereich von 3 bis 4, einer bis zu etwa 75°C erwärmten Laugenlösung oder Mischungen hiervon.

5. Verfahren nach Anspruch 1, **dadurch gekennzeichnet, dass** das inerte Gas Stickstoff ist.

6. Verfahren nach Anspruch 1, **dadurch gekennzeichnet, dass** das inerte Gas Kohlendioxid ist.

7. Verfahren nach Anspruch 1, **dadurch gekennzeichnet, dass** das inerte Gas Argon ist.

8. Verfahren nach Anspruch 1, **dadurch gekennzeichnet, dass** der positive Druck des inerten Gases etwa 10 bis 26 mbar beträgt.

9. Verfahren nach Anspruch 1, **dadurch gekennzeichnet, dass** das sterilisierende Mittel eine Oxoniumlösung aufweist.

10. Verfahren nach Anspruch 1, **dadurch gekennzeichnet, dass** das sterilisierende Mittel eine idophorme Lösung aufweist.

11. Verfahren nach Anspruch 1, **dadurch gekennzeichnet, dass** der Reinigungsschritt (a) die folgenden Schritte aufweist:
a1) Internes Abspülen des stationären Lagertanks oder -behälters (10) mit Trinkwasser;
a2) Aufbringen einer bis zu etwa 75°C erwärmten Laugenlösung auf die inneren Oberflächen des stationären Lagertanks oder -behälters (10); und
a3) internes Abspülen des stationären Lagertanks oder -behälters (10), der im Schritt (a2) mit Trinkwasser behandelt wurde, und Aufbringen eines zweiten Reinigungsmittels wie Phosphorsäurelösung mit einem pH-Wert von etwa 3 bis 4.

12. Verfahren nach Anspruch 1, **dadurch gekennzeichnet, dass** die Schritte des Reinigens und Sterilisierens des stationären Lagertanks oder - behälters (10) durchgeführt werden durch Sprühen von Hochdruckstrahlen gegen die inneren Oberflächen des Behälters (10), wobei die Hochdruckstrahlen von einer Sprühvorrichtung (30) geliefert werden, die intern in dem oberen Bereich des Behälters (10) befestigt ist.

13. Verfahren nach Anspruch 1, **dadurch gekennzeichnet, dass** das zu lagernde sterile Produkt in den stationären Lagertank oder -behälter (10) eingeführt wird, während ein entsprechendes Volumen von inertem Gas entfernt wird.

## Revendications

1. Procédé permettant de stocker de manière aseptique un produit stérile en vrac, dans des réservoirs ou conteneurs (10) de stockage stationnaire conçus pour transporter ledit produit stérile, **caractérisé par** les étapes consistant :
a) à nettoyer le réservoir ou conteneur (10) de stockage stationnaire, en appliquant sur ses surfaces intérieures, au moins un agent nettoyant chimique ;
b) à appliquer un agent stérilisant liquide, au moyen d'un jet continu à haute pression, sur toutes les surfaces intérieures du réservoir ou conteneur nettoyé (10) de stockage stationnaire, de manière telle que le réservoir ou conteneur (10) de stockage stationnaire ne soit pas inondé par ledit agent stérilisant liquide ;
c) à drainer l'agent stérilisant liquide appliqué sur les surfaces intérieures du réservoir ou conteneur (10) de stockage stationnaire ;
d) à remplir complètement d'un liquide stérile le réservoir ou conteneur stérilisé (10) de stockage stationnaire, pour évacuer dans l'atmosphère toute la quantité d'air contenue dans le réservoir ou conteneur ;
e) à sceller et à pressuriser le réservoir ou conteneur (10) de stockage stationnaire avec un gaz inerte, à en évacuer le liquide stérile et à créer une atmosphère de gaz inerte pressurisé à l'intérieur du réservoir ou conteneur (10) de stockage stationnaire ;
f) à fournir une quantité de produit stérile à l'intérieur du réservoir ou conteneur stérilisé de stockage stationnaire, où ledit produit stérile occupe au moins une partie du volume intérieur du réservoir ou conteneur de stockage stationnaire, tout en maintenant l'atmosphère inerte pressurisée ; et
g) à sceller le réservoir ou conteneur stérilisé (10) de stockage stationnaire comprenant le produit stérile, de façon à maintenir l'atmosphère de gaz inerte pressurisé à l'intérieur dudit réservoir ou conteneur.

2. Procédé selon la revendication 1, **caractérisé en ce que** le liquide stérile utilisé au cours de l'étape (c) est de l'eau pasteurisée.

3. Procédé selon la revendication 1, **caractérisé en ce que** le produit stérile en vrac comprend un jus de fruit.

4. Procédé selon la revendication 1, **caractérisé en ce que** l'agent nettoyant chimique est sélectionné parmi le groupe se composant d'une solution d'acide phosphorique ayant un pH compris entre 3 et 4, d'une solution de soude caustique chauffée à 75°C environ ou bien de mélanges de ces solutions.

5. Procédé selon la revendication 1, **caractérisé en ce que** le gaz inerte est de l'azote.

6. Procédé selon la revendication 1, **caractérisé en ce que** le gaz inerte est du dioxyde de carbone.

7. Procédé selon la revendication 1, **caractérisé en ce que** le gaz inerte est de l'argon.

8. Procédé selon la revendication 1, **caractérisé en ce que** la pression positive du gaz inerte est comprise entre 10 mbar et 26 mbar environ.

9. Procédé selon la revendication 1, **caractérisé en ce que** l'agent stérilisant comprend une solution d'oxonia.

10. Procédé selon la revendication 1, **caractérisé en ce que** l'agent stérilisant comprend une solution d'iodoforme.

11. Procédé selon la revendication 1, **caractérisé en ce que** l'étape de nettoyage (a) comprend les étapes suivantes consistant :
a1) à rincer intérieurement, avec de l'eau potable, le réservoir ou conteneur (10) de stockage stationnaire ;
a2) à appliquer une solution de soude caustique chauffée à 75°C environ, sur les surfaces intérieures du réservoir ou conteneur (10) de stockage stationnaire ; et
a3) à rincer intérieurement, avec de l'eau potable, le réservoir ou conteneur (10) de stockage stationnaire traité au cours de l'étape (a2) et à appliquer un deuxième agent nettoyant tel qu'une solution d'acide phosphorique ayant un pH compris entre 3 et 4 environ.

12. Procédé selon la revendication 1, **caractérisé en ce que** les étapes de nettoyage et de stérilisation du réservoir ou conteneur (10) de stockage stationnaire sont effectuées par pulvérisation de jets à haute pression contre les surfaces intérieures dudit conteneur (10), où lesdits jets à haute pression sont fournis par un dispositif de pulvérisation (30) monté à l'intérieur, au niveau de la partie supérieure du conteneur (10).

13. Procédé selon la revendication 1, **caractérisé en ce que** le produit stérile devant être stocké est introduit dans le réservoir ou conteneur (10) de stockage stationnaire, tandis qu'un volume correspondant de gaz inerte est éliminé.
